# EUROPEAN PATENT APPLICATION

(11) **EP 1 344 547 A1**
(43) Date of publication of application: **17.09.2003**
(21) Application number: 02005515.8
(22) Date of filing: 11.03.2002
(51) Int. Cl.: A61M 25/00

(54) **Method of forming a balloon of a balloon catheter**

(71) Applicant: JOMED NV, 6235 NS Ulestraten (NL)
(72) Inventor: Boelens, Johan, 72379 Hechingen (DE); Trösken, Volker, 72379 Hechingen (DE)
(74) Representative: Schmitz, Hans-Werner, Dipl.-Ing.

(57) **Abstract**

In order to produce a balloon catheter having a balloon with an extremely low profile, a method of forming a balloon of a balloon catheter is proposed, comprising the following method steps:
a) introducing a tube (5) of balloon material into a balloon forming mold (1);
b) forming the tube (5) into the balloon shapes;
c) taking the balloon out of the mold (1);
d) folding the balloon;
e) wrapping a thread around the folded balloon; and
f) removing the thread from the balloon after a suitable period of time.

## Description

The present invention concerns a method of forming a balloon of a balloon catheter.

Balloon catheters are used for recanalisation of blocked or narrowed blood vessels. For this purpose, a known balloon catheter comprises an expandable balloon. The balloon is folded and a stent may be crimped onto the folded balloon for introducing the balloon catheter and the stent crimped thereon into the body vessel. Alternatively, only a balloon may be used for widening a stenosis of the blood vessel.

Known balloon catheters, however, often cause problems in case of extremely narrow vessels as the balloon of known balloon catheters cannot be folded to extremely low profiles and diameters, respectively.

It is therefore an object of the present invention to provide a method of forming a balloon of a balloon catheter that makes it possible to produce a balloon that can be folded to extremely low profiles.

The solution of this object is achieved by the features of claim 1.

By wrapping a thread or flatband around the folded balloon and leaving that thread on the folded balloon for a predetermined period of time, it is possible to compress the folds tightly together what results on the one hand in an extremely small profile of the balloon in its folded condition, and on the other hand in a somewhat rough surface of the folded balloon resulting in a higher surface friction. This, in turn, results in the advantage that the stent crimped onto the folded balloon can temporarily be fixed much easier on the balloon, thus preventing the stent from slipping from the balloon when being moved within a body vessel. If only a balloon catheter without a stent shall be produced, it is preferable to use a flat-shaped thread or band.

For a pre-mounted system (balloon and stent), the use of a round or otherwise shaped thread is preferable.

Moreover, the method according to the present invention results in a balloon that can be easily pushed forward through extremely curved vessels without causing the problem that the folds open when the balloon moves through such extreme curves.

Moreover, also the conical end portions of the balloon, which are stiffer or harder than the central portion of the balloon, can be compressed more easily with the method according to the present invention.

The dependent claims contain advantageous embodiments of the method according to claim 1.

So, the folded balloon can be somewhat heated when the thread is wrapped around the folded balloon, thus increasing or accelerating the effect of the higher compression.

A preferred material of the thread is polytetrafluorethylen. Moreover, any other material is possible and any shape of thread profile or band profile can be used.

The method according to the present invention can be effected manually or automatically by a suitable machine.

Claim 6 defines a balloon catheter that is produced according to the principles of the method according to the present invention.

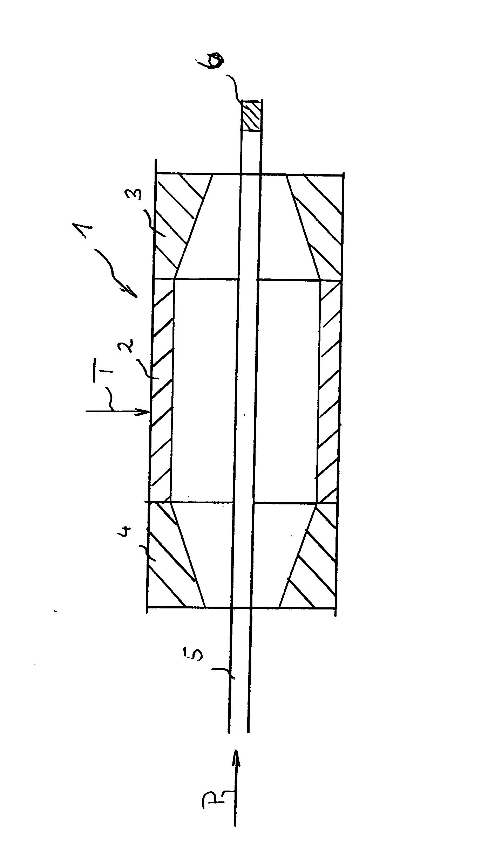

Further features, advantages and effects according to the present invention will become apparent from the accompanying drawings. The single figure of the drawings shows an extremely simplified schematic presentation of a mold 1 for forming the balloon of a balloon catheter. The mold comprises a central cylindrical portion 2 and two conical end portions 3 and 4.

The inner shape of these three portions 2, 3 and 4, that is apparent from the figure, corresponds to the form of an expanded balloon of a balloon catheter for placing a stent.

For forming the balloon, a cube 5 is introduced into the mold 1. The tube 5 is closed at one end 6. Thereafter, pressure P is applied to the closed tube 5, thus expanding the tube 5 into the form corresponding to the inner shape of the three portions 2, 3 and 4 of the mold 1. During this expansion, the mold 1 is usually heated, what has been symbolized in the figure by the arrow T.

After having formed tube 5 into the balloon shape, the balloon is removed from the mold 1 and then the balloon may be folded. After folding the balloon, a thread, preferably made out of PTFE, is wrapped around the folded balloon, thereby compressing the folds together.

After leaving the thread on the folds of the balloon, the thread is removed so that the balloon is ready or can be used for being combined with a stent that is crimped onto the folded balloon portion.

For the sake of completeness, it should be noted that forming a balloon of a balloon catheter also includes attaching the balloon with the respective tubing of the catheter system, preferably before wrapping the thread around the balloon.

## Claims

1. Method of forming a balloon of a balloon catheter, comprising the following method steps:
a) wrapping a thread or flat band around the balloon;
b) leaving the thread or flat band on the balloon for a suitable period of time;
c) removing the thread or flat band from the balloon after a suitable period of time.

2. Method according to claim 1, being **characterized by** heating the folded balloon during method step a) and/or b).

3. Method according to claim 1 or 2, **characterized by** using polytetrafluorethylene, polyurethane or any other suitable material for the thread or flat band.

4. Method according to one of claims 1 to 3, being **characterized by** folding the balloon prior to steps a) to c).

5. Method according to one of claims 1 to 4, being **characterized by** the following method steps prior to steps a) to c):
d) introducing a tube (5) of balloon material into a balloon forming mold (1);
e) forming the tube (5) into the balloon shape;
f) taking the balloon out of the mold (1).

6. Method according to one of claims 1 to 5, being **characterized by** attaching the balloon to a catheter tubing.

7. Method according to one of claims 1 to 6 being **characterized by** effecting the method steps of claims 1 and 4 to 6 manually.

8. Method according to one of claims 1 to 6, being **characterized by** effecting the method steps of claims 1 and 4 to 6 by a machine.

9. Balloon catheter having a foldable and expandable balloon being **characterized by** a method according to one of claims 1 to 8.
